Europäisches Patentamt

European Patent Office

Office européen des brevets

(11) Numéro de publication: **0 028 198**
**A1**

## (12) DEMANDE DE BREVET EUROPEEN

(21) Numéro de dépôt: **80420111.9**

(22) Date de dépôt: **13.10.80**

(51) Int. Cl.³: **A 61 M 1/03**, F 16 L 37/02

---

(30) Priorité: **16.10.79 FR 7926105**

(43) Date de publication de la demande: **06.05.81**
**Bulletin 81/18**

(84) Etats contractants désignés: **AT BE CH DE GB IT LI LU NL SE**

(71) Demandeur: **Laboratoire AGUETTANT, 1, avenue Jules Carteret, Lyon 7ème, Rhône (FR)**

(72) Inventeur: **Aguettant, Georges, 1, avenue Jules Carteret, Lyon 7ème, Rhône (FR)**

(74) Mandataire: **Maureau, Bernard, Cabinet GERMAIN & MAUREAU Le Britannia - Tour C 20, Boulevard Eugène Déruelle, F-69003 Lyon (FR)**

---

(54) **Dispositif de connexion avec étanchéité entre deux embouts en vue de la mise en communication de deux volumes.**

(57) Dans ce dispositif, chaque embout (2, 4) est entouré par une jupe (7, 14) de forme sensiblement cylindrique raccordée à une collerette (6, 13) solidaire de l'embout (2, 4) considéré et permettant le passage de celui-ci, la collerette (6, 13) étant située en retrait par rapport à l'extrémité de connexion de l'embout (2, 4) qui est elle-même en retrait par rapport au bord de la jupe (7, 14), le diamètre de la jupe (7) entourant un embout (2) étant différent du diamètre de la jupe (14) entourant l'autre embout (4), chaque embout (2, 4) étant associé à un bouchon comportant un fond à partir duquel s'étend une jupe de forme générale cylindrique, apte à être engagée avec étanchéité sur la jupe (7, 14) entourant ledit embout (2, 4).

Application aux connexions réalisées dans le domaine médical.

La présente invention a pour objet un dispositif de connexion avec étanchéité entre deux embouts en vue de la mise en communication de deux volumes, et plus spécialement un dispositif pour connexions à usage médical devant être réalisées dans de bonnes conditions de stérilité.

Dans le domaine médical, il est impératif de réaliser les connexions entre tubulures par exemple pour perfusions ou transfusions dans des conditions évitant au maximum la contamination du patient par le liquide transféré.

L'importance d'une excellente stérilité des connexions se pose particulièrement en matière de dialyse péritonéale. Cette technique permet de réaliser l'épuration du sang d'un individu non pas par passage du sang dans un appareil de dialyse, mais par réalisation de l'opération de dialyse au niveau du péritoine du patient, qui joue le rôle de membrane de dialyse, après introduction dans le volume délimité par le péritoine d'une certaine quantité de soluté.

Une première tubulure débouchant dans le péritoine et traversant les différentes parois musculaires du ventre du patient, est installée de manière durable. Sur cette tubulure est montée une ligne de plusieurs dizaines de centimètres de longueur qui demeure en place pendant plusieurs semaines et qui est mise en place et retirée sous contrôle médical. Les risques de contamination à ce niveau sont donc limités. L'extrémité libre de la ligne précitée sert à la connexion avec le récipient contenant le liquide de dialyse pur et qui est également destiné à recevoir le liquide souillé après dialyse, ce récipient pouvant être constitué par exemple par une poche souple en matière synthétique.

D'un point de vue pratique, le patient connecte lui-même à la ligne une poche remplie de liquide de dialyse pur, puis laisse se réaliser le transfert à l'intérieur du péritoine d'un volume de l'ordre de deux litres, ceci pour une personne de corpulence normale. La poche est alors déconnectée de la ligne, après quoi l'individu peut vaquer à ses occupations. En fin de dialyse, au bout de deux heures environ, la poche vide est reconnectée à la ligne afin de recueillir le liquide de dialyse souillé. Une telle succession d'opérations doit être réalisée quatre fois par jour.

L'intérêt de la dialyse péritonéale réside dans la réalisation du traitement avec une grande efficacité, sans nécessiter un matériel important, au domicile même du patient, sans surveillance médicale permanente.

Il ressort du mode opératoire décrit précédemment que chaque connexion entre la ligne et la poche contenant le liquide pur ou destiné à recevoir le liquide souillé, doit être effectuée dans des conditions de stérilité parfaite, pour éviter toute contamination se traduisant très rapidement par une péritonite. Les risques de contamination sont en effet importants du fait que les solutés utilisés pour les échanges sont des milieux physiologiques favorisant la prolifération des germes, le changement de récipient étant effectué 4 à 5 fois par jour. Par ailleurs, le patient souffrant déjà d'insuffisance rénale a des capacités de défense immunitaire limitées.

La présente invention vise à pallier les lacunes de la technique existante en fournissant un dispositif de connexion de deux embouts dans des conditions stériles, pouvant satisfaire parfaitement à la dialyse péritonéale.

Le dispositif de connexion qu'elle concerne est du type comportant deux embouts solidaires chacun d'une tubulure ou d'un récipient, ces deux embouts complémentaires étant de type connu, par exemple de type Luer, et permettant de réaliser une connexion avec étanchéité pour la mise en communication de deux volumes.

Selon la caractéristique essentielle de l'invention, chaque embout est entouré par une jupe de forme sensiblement cylindrique raccordée à une collerette solidaire de l'embout considéré et permettant le passage de celui-ci, la collerette étant située en retrait par rapport à l'extrémité de connexion de l'embout qui est elle-même en retrait par rapport au bord de la jupe, le diamètre de la jupe entourant un embout étant différent du diamètre de la jupe entourant l'autre embout, chaque embout étant associé à un bouchon comportant un fond à partir duquel s'étend une jupe de forme générale cylindrique, apte à être engagée avec étanchéité sur la jupe entourant ledit embout.

L'intérêt de cet agencement est que chaque bouchon peut recevoir une certaine quantité d'antiseptique et former, avec la jupe de l'embout qui lui est associé, un réservoir assurant le maintien de cet embout en conditions stériles.

Il est à noter que les différentes jupes sont avantageusement de forme légèrement conique, ceci pour des simplicités de réalisation si elles sont en matière synthétique et afin de permettre des emboîtements avec étanchéité.

L'utilisation de ce dispositif est la suivante, dans la mesure où l'on considère que le péritoine du patient contient un certain volume de soluté et qu'il convient de commencer le cycle par un drainage :

- déboucher la poche vide pour le drainage,
- déboucher la ligne et poser le bouchon associé à celle-ci, de telle sorte que son fond repose sur une surface propre, la jupe étant tournée vers le haut,
- connecter la ligne et la poche,
- poser la poche vide à un niveau inférieur à celui du péritoine du patient,
- ouvrir la pince fermant la ligne,
- laisser le soluté se drainer.

Pendant le temps de drainage, il convient de procéder aux opérations suivantes :

- remplir le bouchon de la ligne avec un antiseptique,
- prendre une poche neuve,
- la sortir de son emballage et la mirer,
- y ajouter éventuellement, stérilement des médicaments.

Après que le drainage soit terminé, il convient de :

- fermer la pince associée à la ligne,

- clamper la poche pleine de liquide drainé,

- déboucher la poche neuve,

- déconnecter la poche usée,

- connecter la poche neuve et la ligne,

- casser l'obturateur de la poche s'il y en a un,

- ouvrir la pince de la ligne,

- suspendre la poche à un niveau supérieur à celui du péritoine et laisser l'injection de dialysat dans celui-ci se produire. Au cours de l'injection, le patient remplit le bouchon de la poche avec un antiseptique. Puis lorsque l'injection est terminée, il convient de procéder aux opérations suivantes :

- fermer la pince de la ligne,

- déconnecter la poche et la ligne,

- reboucher la ligne avec son bouchon rempli d'antiseptique,

- reboucher la poche avec son bouchon rempli d'antiseptique,

- replier la ligne,

- ranger la poche jusqu'au prochain drainage.

Ce dispositif présente l'intérêt de :

- garantir la stérilité d'une connexion au fil des jours malgré les nombreuses manipulations,

- simplifier les gestes nécessaires pour adapter stérilement un récipient et une tubulure,

- permettre à une personne dénuée de formation médicale d'assurer sur elle-même une thérapeutique répétitive,

- être très simple de réalisation et, par conséquent, d'un prix de revient peu élevé.

Selon une autre caractéristique de l'invention, le diamètre extérieur de la jupe de plus faible section entourant un embout est sensiblement égal au diamètre intérieur de la jupe entourant l'autre embout.

Cet agencement est intéressant en ce sens que, lors de l'emboîtement des deux embouts, est également réalisé un emboîtement avec étanchéité des deux jupes. Il est donc ainsi ménagé un volume annulaire étanche entre les deux embouts et les deux jupes en position connectée, ce qui favorise les conditions de stérilité de la connexion.

Avantageusement, le diamètre du fond de chaque bouchon est supérieur au diamètre moyen de la jupe qu'il comporte. Ceci assure une bonne assise aux bouchons lorsque ceux-ci sont posés par leurs fonds sur une surface horizontale en période de remplissage par un antiseptique.

De toute façon, l'invention sera bien comprise à l'aide de la description qui suit en référence au dessin schématique annexé représentant, à titre d'exemple non limitatif, une forme d'exécution de ce dispositif de connexion :

Figure 1 est une vue en perspective d'un premier embout et du bouchon qui lui est associé ;

Figure 2 est une vue en perspective du second embout et du bouchon qui lui est associé ;

Figures 3 et 4 sont deux vues en coupe longitudinale des deux embouts, chacun étant équipé de son bouchon ;

Figure 5 est une vue en coupe longitudinale des deux embouts connectés.

Le dispositif selon l'invention comporte, dans sa forme d'exécution décrite au dessin, deux embouts à conicité Luer à savoir un embout femelle 2 relié à une tubulure 3 débouchant dans la poche contenant le dialysat, et un embout male 4 relié à la ligne 5 associée au patient, dans le cas d'une connexion pour une dialyse péritonéale.

Sur l'embout 2 est montée, en retrait de l'extrémité de celle-ci, une collerette 6 à partir de laquelle s'étend une jupe 7 sensiblement cylindrique dont le bord est situé au-delà de l'embout 2. A l'embout 2 est également associé un bouchon 8 comportant un fond 9 et une jupe sensiblement cylindrique 10, de diamètre tel qu'elle puisse venir s'emboîter avec étanchéité sur la jupe 7. Du fond 9 fait saillie une partie 12 destinée à former un obturateur pénétrant dans l'embout femelle 2 en position montée du bouchon 8. Le diamètre du fond 9 est sensiblement supérieur à celui de la jupe 10 de manière à assurer une bonne stabilité au bouchon quand il est posé sur un plan horizontal et est rempli d'antiseptique.

A l'embout mâle 4 est associée une collerette 13 située en retrait de l'extrémité de celui-ci à partir de laquelle s'étend une jupe 14 de forme générale cylindrique entourant l'embout 4 et dont le bord est situé au-delà de l'extrémité de celui-ci. Cet embout 4 peut être équipé d'un bouchon 15 comportant un fond 16 à partir duquel s'étend une jupe de forme générale cylindrique 17, de diamètre intérieur correspondant au diamètre extérieur de la jupe 14, de manière à pouvoir s'emboîter avec étanchéité sur celle-ci. Du fond 16 du bouchon 15 font saillie deux éléments concentriques 18 et 19 destinés, en position montée du bouchon, à être placés respectivement à l'intérieur et à l'extérieur de l'embout mâle.

Il est à noter que le diamètre du fond 16 est sensiblement supérieur à celui de la jupe 17, de manière à procurer une bonne stabilité au bouchon lorsqu'il est posé sur une surface horizontale et est rempli d'antiseptique.

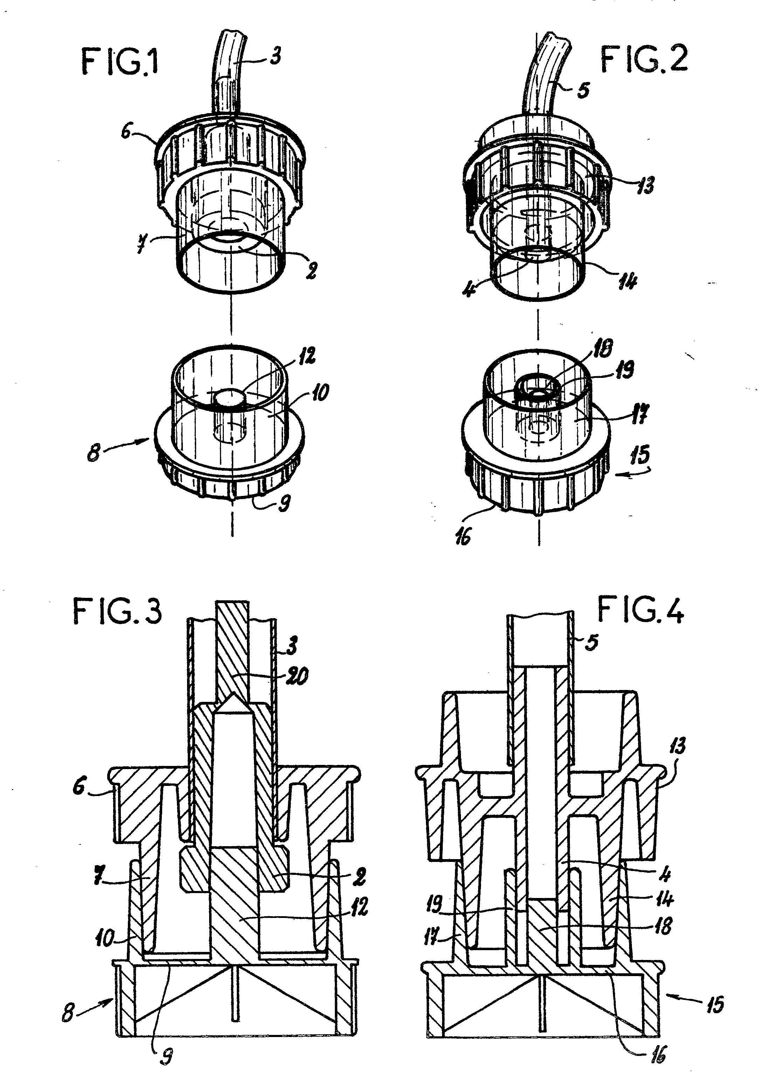

Les figures 3 et 4 sont des vues en coupe représentant les deux embouts équipés de leurs bouchons respectifs, la poche se trouvant remplie de liquide pur puisque l'obturateur 20, qui lui est associé, n'a pas encore été brisé.

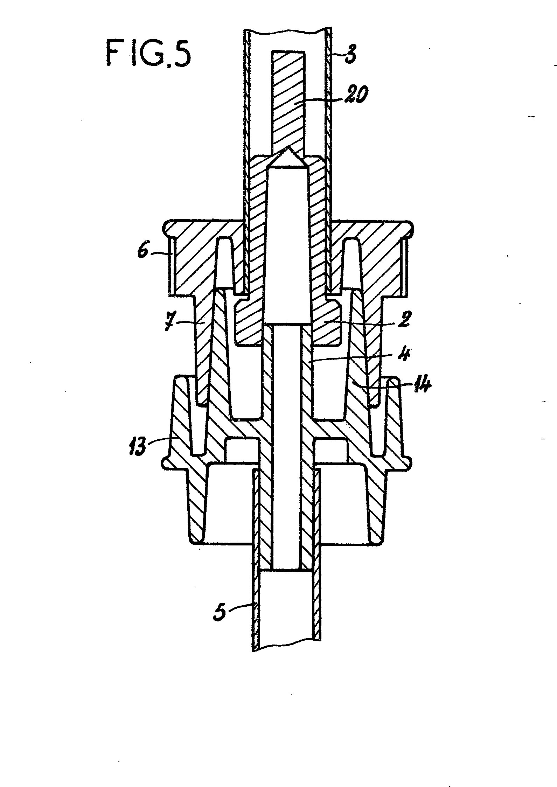

La figure 5 représente les deux embouts en position connectée. Il ressort du dessin que l'étanchéité est réalisée non seulement au niveau des embouts 2 et 4 proprement dits, mais également entre les jupes 7 et 14 associées à ceux-ci, le diamètre extérieur de la jupe 14 correspondant au diamètre intérieur de la jupe 7.

En ce qui concerne la mise en place des bouchons 8 et 15 sur leurs embouts respectifs, après qu'ils aient été remplis de liquide antiseptique , il est à noter qu'elle se fait, le bouchon posé sur une surface horizontale, par engagement de la jupe associée à l'embout considéré à l'intérieur de la jupe du bouchon.

Comme il ressort de ce qui précède, l'invention apporte une grande amélioration à la technique existante en fournissant un dispositif de connexion dans lequel chaque embout se trouve à l'intérieur d'une enceinte qui, pouvant être bouchée par un bouchon clos, peut être remplie d'une solution antiseptique et donc être conservée en conditions stériles entre les manipulations.

Comme il va de soi, l'invention ne se limite pas à la seule forme d'exécution de ce dispositif de connexion, décrite ci-dessus à titre d'exemple ; elle en embrasse, au contraire, toutes les variantes de réalisation.

0028198

8

## - REVENDICATIONS -

1. - Dispositif de connexion avec étanchéité entre deux embouts, du type comportant deux embouts (2, 4) solidaires chacun d'une tubulure (3, 5) ou d'un récipient, ces deux embouts complémentaires étant de type connu, par exemple de type Luer, et permettant de réaliser une connexion avec étanchéité pour la mise en communication de deux volumes, caractérisé en ce que chaque embout est entouré par une jupe (7, 14) de forme sensiblement cylindrique raccordée à une collerette (6, 13) solidaire de l'embout considéré et permettant le passage de celui-ci, la collerette (6, 13) étant située en retrait par rapport à l'extrémité de connexion de l'embout (3, 5), qui est elle-même en retrait par rapport au bord de la jupe (7, 14), le diamètre de la jupe entourant un embout étant différent du diamètre de la jupe entourant l'autre embout, chaque embout étant associé à un bouchon (8, 15) comportant un fond (9, 16) à partir duquel s'étend une jupe (10, 17) de forme générale cylindrique, apte à être engagée avec étanchéité sur la jupe (7, 14) entourant ledit embout.

2. - Dispositif selon la revendication 1, caractérisé en ce que le diamètre extérieur de la jupe (14) de plus faible section entourant un embout (5) est sensiblement égal au diamètre intérieur de la jupe (7) entourant l'autre embout (3).

3. - Dispositif selon l'une quelconque des revendications 1 et 2, caractérisé en ce que le diamètre du fond (9, 16) de chaque bouchon est supérieur au diamètre moyen de la jupe (7, 14) qu'il comporte.

0028198

FIG.1

FIG.2

FIG.3

FIG.4

FIG.5

**0028198**

Numéro de la demande

**RAPPORT DE RECHERCHE EUROPEENNE**

Office européen
des brevets

EP 80 42 0111.9

## DOCUMENTS CONSIDERES COMME PERTINENTS

| Catégorie | Citation du document avec indication, en cas de besoin, des parties pertinentes | Revendication concernée |
|---|---|---|
| | CH - A - 456 050 (KAUTEX-WERK) <br> * fig. 2, 3 * | 1 |
| | -- | |
| | FR - A - 1 234 323 (B. BRAUN) <br> * fig. 3, 4 * | 1 |
| | -- | |
| A | DE - B2 - 2 415 007 (UNION CARBIDE) | |
| | -- | |
| A | FR - A - 2 156 567 (PRO MEDICAL ENGI-NEERING) | |
| | -- | |
| A | FR - A - 1 307 306 (G.A.J.J. GUIRAUD) | |
| | -- | |
| P | DE - A1 - 2 853 635 (K. AFFELD) <br> * revendication 1; fig. 1 * | 1 |
| | ---- | |

**CLASSEMENT DE LA DEMANDE (Int. Cl.²)**

A 61 M  1/03

F 16 L 37/02

**DOMAINES TECHNIQUES RECHERCHES (Int. Cl.²)**

A 61 M  1/00

F 16 L 37/00

F 16 L 55/00

**CATEGORIE DES DOCUMENTS CITES**

X: particulièrement pertinent
A: arrière-plan technologique
O: divulgation non-écrite
P: document intercalaire
T: théorie ou principe à la base
   de l'invention
E: demande faisant interférence
D: document cité dans
   la demande
L: document cité pour d'autres
   raisons

&: membre de la même famille,
   document correspondant

Le présent rapport de recherche a été établi pour toutes les revendications

| Lieu de la recherche <br> Berlin | Date d'achèvement de la recherche <br> 14-01-1981 | Examinateur <br> SCHLABBACH |
|---|---|---|

OEB Form 1503.1  06.78